Europäisches Patentamt

**European Patent Office**

Office européen des brevets

(19)

(11) Veröffentlichungsnummer: **0 038 409**
**B1**

(12)

# EUROPÄISCHE PATENTSCHRIFT

(45) Veröffentlichungstag der Patentschrift:
02.05.85

(21) Anmeldenummer: **81101248.3**

(22) Anmeldetag: **21.02.81**

(51) Int. Cl.⁴: **C 07 C 89/02,** C 07 C 91/10

(54) Verfahren zur Herstellung von 1-Amino-propandiol-(2,3) (II).

(30) Priorität: **12.04.80 DE 3014109**

(43) Veröffentlichungstag der Anmeldung:
**28.10.81 Patentblatt 81/43**

(45) Bekanntmachung des Hinweises auf die Patenterteilung:
**02.05.85 Patentblatt 85/18**

(84) Benannte Vertragsstaaten:
**BE CH DE FR GB LI NL**

(56) Entgegenhaltungen:
**DE - C - 694 992**
**FR - A - 1 432 428**
**US - A - 3 544 632**

**JOURNAL OF ORGANIC CHEMISTRY, Band 27, Nr. 6,
Juni 12, 1962 K. BAUM et al.: "The Mannich
Condensation of 3-Amino-1,2-propanediol with
2,2-Dinitropropanol and the Nitration of the Product",
Seiten 2231-2233**

(73) Patentinhaber: **Degussa Aktiengesellschaft,
Weissfrauenstrasse 9, D-6000 Frankfurt am Main 1 (DE)**

(72) Erfinder: **Kleemann, Axel, Dr., Greifenhagenstrasse 25,
D-6450 Hanau 9 (DE)**
Erfinder: **Nygren, Robert, Dr., Fürstenbergstrasse 8,
D-6450 Hanau 9 (DE)**
Erfinder: **Wagner, Rudolf, Dr., Greifenhagenstrasse 9,
D-6450 Hanau 9 (DE)**

## Beschreibung

Die Herstellung von 1-Amino-propandiol-(2,3) durch Addition von Ammoniak an Glycid wurde erstmals von L. Knorr u. E. Knorr (Ber. deutsch. Chem. Ges. 32, 750 (1899)) beschrieben. Die Autoren setzten hierbei einen Gewichtsteil Glycid mit 100 Gewichtsteilen 25%igem wässrigem Ammoniak ein und erhielten dann nach destillativer Aufarbeitung 1-Amino-propandiol-(2,3) in einer Ausbeute von 44%, bezogen auf eingesetztes Glycid. Das Gewichtsverhältnis Glycid zu wässrigem Ammoniak (25%ig) = 1:100 bedeutet ein Molverhältnis Glycid zu Ammoniak = 1:109.

Diese Herstellmethode von 1-Amino-propandiol-(2,3) wurde von K. Baum und W.T. Maurice (J. Org. Chem. 27, 2231 (1962)) überprüft, wobei dann bei denselben Bedingungen eine Ausbeute von 68% der Theorie erzielt werden konnte. Diese bessere Ausbeute findet ihre Begründung darin, dass die erstgenannten Autoren das Reaktionsprodukt bei 235–250 °C/320 mm Hg destillierten, während die zuletzt genannten Autoren schonender, nämlich bei 80–106 °C/0,1–0,15 mm Hg destillierten und somit keine Verluste durch thermische Zersetzung verursachten.

Wenn auch das zuletzt genannte Verfahren bessere Ausbeuten gegenüber dem Verfahren von Knorr (loc. cit.) bringt, so stellen die im Kreis zu führenden Mengen an wässrigem Ammoniak bei der technischen Durchführung eine erhebliche Belastung dar.

Ausserdem wird durch sie ein sehr grosser Reaktionsraum nötig auf Grund des oben genannten Molverhältnisses von Glycid zu Ammoniak, sowie eine Destillationsanlage zum Konzentrieren der im Kreis zu führenden verdünnten wässrigen Ammoniaklösung.

Wenn es auch nach der US-PS 3 544 632 bekannt ist, Alkanolamine durch Umsetzung von Alkylenoxiden mit Kohlenwasserstoffen und flüssigem Ammoniak in wässrigem Medium umzusetzen, so handelt es sich hier um die Reaktion von monofunktionellen Epoxiden mit Ammoniak, die nicht zur Selbstkondensation neigen.

Zwar wird ein diskontinuierliches Verfahren zur Herstellung der gleichen Stoffe in der FR-PS 1 423 428 beschrieben, bei denen aliphatische Epoxyalkohole eingesetzt werden. Glycid als Anfangsglied wird aber nicht genannt, und bei den in der Patentschrift verwendeten Verbindungen besteht an sich keine Gefahr einer Eigenkondensation. Das Verfahren lässt sich ausserdem nur diskontinuierlich durchführen und ist langwierig.

Zweck des erfindungsgemässen Verfahrens ist daher ein Verfahren zur Herstellung von 1-Amino-propandiol-(2,3) in guten Ausbeuten und auf technisch einfache Weise.

Es wurde nun gefunden, dass man die Umsetzung von Glycid mit Ammoniak in homogener flüssiger Phase mit sehr guten Ausbeuten und ohne besonderen technischen Aufwand durchführen kann, wenn man Glycid und flüssiges Ammoniak unter einem solchen Druck, dass das Ammoniak flüssig bleibt, in Gegenwart einer geringen Menge Wasser miteinander umsetzt.

Das Molverhältnis von Glycid zu flüssigem Ammoniak liegt im allgemeinen im Bereich von 1:5 bis 1:20.

Bevorzugt ist ein Molverhältnis von 1:10 bis 20, ganz bevorzugt ein Verhältnis von 1:7.

Eine Erhöhung des Molverhältnisses über 1:20 hinaus bringt keine erheblichen Ausbeutesteigerungen mehr; unter einem Molverhältnis von 1:5 ist nur noch die technisch gegenüber dem Stand wesentlich vereinfachte Durchführung interessant.

Der Ausdruck «geringe Wassermenge» ist relativ zu dem bisherigen Stand der Technik mit seinen sehr grossen, im Kreis zu führenden Wassermengen zu verstehen (loc. cit.).

Das Gewichtsverhältnis von Glycid zu Wasser liegt in dem Bereich von 1:0,05 bis 1:10, bevorzugt ist der Bereich von 1:1. Das Gewichtsverhältnis von Glycid zu Wasser kann auch oberhalb 10 liegen. Je mehr dieses Verhältnis oberhalb 1:10 liegt, desto geringer ist aber die Raumzeitausbeute. Der Druckbereich liegt zwischen 5 bis 150 bar, bevorzugt bei 20 bis 90 bar.

Als Reaktionstemperaturen werden 20 °C bis 180 °C, bevorzugt 50 °C bis 120 °C verwandt.

Temperaturen oberhalb und unterhalb des genannten Bereiches sind möglich, führen aber bei Temperaturen unterhalb des genannten Bereiches zu unvertretbar langen Reaktionszeiten und bei solchen oberhalb des Bereiches zur stärkeren Nebenproduktbildung.

Der technische Fortschritt des erfindungsgemässen Verfahrens, das sowohl diskontinuierlich wie kontinuierlich durchgeführt werden kann, liegt einmal in den grossen Ausbeutesteigerungen, die bei dem bevorzugten Gewichtsverhältnis von Glycid zu Wasser von 1:1 zu einer bisher nicht bekannten hohen Ausbeute führten.

Hinzu kommt, dass nicht nur die Ausbeute an sich, sondern auch die Raum-Zeitausbeute wesentlich erhöht wurde und zwar gegenüber dem Verfahren von Baum und Maurice (loc. cit.) um den Faktor 50.

Ausserdem ist der technische Aufwand sehr verringert worden, da nicht mehr die erheblichen Wassermengen rezykliert bzw. die sehr verdünnte wässrige Ammoniaklösung konzentriert werden muss.

Es war nicht vorauszusehen, dass die Anwesenheit der vergleichsweise geringen Wassermengen die Selektivität der Umsetzung von Glycid und flüssigem Ammoniak unter Druck erhöhen würde.

Das erfindungsgemässe Verfahren wird anhand der folgenden Beispiele näher erläutert:

Versuchsapparatur

Die nachstehenden Beispiele wurden in folgender Versuchsapparatur durchgeführt:

Aus einer mit Glycid bzw. Glycid/Wasser gefüllten Vorlage und einer Druckflasche mit flüssigem Ammoniak wurden mittels zweier Pumpen mengengeregelt die Reaktanten in den Reaktor gefördert. Dieser bestand aus einem Doppelmantelrohr, wobei der äussere Mantelraum dazu diente, mit Hilfe von Wasser das Reaktionsgemisch im inneren Rohr auf die gewünschte Temperatur zu bringen und die Reaktionswärme abzuführen. Die Reaktion wurde in flüssiger homogener Phase durchgeführt. Der zur Flüssighaltung der Reaktanten nötige Druck wurde durch ein Druckhalteventil am Ende des Doppelmantelrohres gehalten. Das innere Rohr, also die Reaktionszone, hatte ein Volumen von 4,2 Ltr. Nach Durchlaufen der Reaktionsstrecke wurde das Reaktionsgemisch am Druckregelventil entspannt und in eine Vorlage geleitet. Aus dem so erhaltenen Rohprodukt wurde durch fraktionierte Vakuumdestillation das 1-Aminopropandiol-(2,3) isoliert.

### Beispiel 1

In den oben genannten Reaktor wurden bei 85°C und 43 bar pro Stunde 0,5 kg Glycid, 0,5 kg Wasser und 1,7 kg flüssiges Ammoniak eindosiert. (Molverhältnis von Glycid zu Ammoniak = 1:16,8, Gewichtsverhältnis von Glycid zu Wasser = 1:1).

Nach destillativer Aufarbeitung des Reaktionsproduktes erhielt man 0,51 kg Aminopropandiol pro Stunde, entsprechend 83% der Theorie, bezogen auf eingesetztes Glycid. Siedepunkt = 94°C (0,2 Torr), Reinheit $\geqq$ 99,5 Gew.-% (Amintitration).

### Beispiel 2

In den oben genannten Reaktor wurden bei 75°C und 30 bar pro Stunde 0,6 kg Glycid, 0,6 kg Wasser und 1,4 kg flüssiges Ammoniak eindosiert. (Molverhältnis von Glycid zu Ammoniak = 1:10,3, Gewichtsverhältnis von Glycid zu Wasser = 1:1).

Nach destillativer Aufarbeitung des Reaktionsproduktes erhielt man 0,55 kg Aminopropandiol pro Stunde, entsprechend 75% der Theorie, bezogen auf eingesetztes Glycid.

### Beispiel 3

In den oben genannten Reaktor wurden bei 85°C und 43 bar pro Stunde 0,75 kg Glycid, 0,05 kg Wasser und 2,6 kg flüssiges Ammoniak eindosiert. (Molverhältnis von Glycid zu Ammoniak = 1:15, Gewichtsverhältnis von Glycid zu Wasser = 1:0,07).

Nach destillativer Aufarbeitung des Reaktionsproduktes erhielt man 0,64 kg Aminopropandiol pro Stunde, entsprechend 69% der Theorie, bezogen auf eingesetztes Glycid.

### Beispiel 4

In den oben genannten Reaktor wurden bei 85°C und 43 bar pro Stunde 0,5 kg Glycid, 2,0 kg Wasser und 1,7 kg flüssiges Ammoniak eindosiert. (Molverhältnis von Glycid zu Ammoniak = 1:16,8, Gewichtsverhältnis von Glycid zu Wasser = 1:4).

Nach destillativer Aufarbeitung des Reaktionsproduktes erhielt man 0,49 kg Aminopropandiol pro Stunde, entsprechend 79% der Theorie, bezogen auf eingesetztes Glycid.

### Beispiel 5

In den oben genannten Reaktor wurden bei 85°C und 43 bar pro Stunde 0,75 kg Glycid, 0,25 kg Wasser und 2,6 kg flüssiges Ammoniak eindosiert (Molverhältnis von Glycid zu Ammoniak = 1:15, Gewichtsverhältnis von Glycid zu Wasser = 1:0,3).

Nach destillativer Aufarbeitung des Reaktionsproduktes erhielt man 0,69 kg Aminopropandiol pro Stunde, entsprechend 74,2% der Theorie, bezogen auf eingesetztes Glycid.

Die Siedepunkte und Reinheit des Produktes in den Beispielen 2–5 entsprachen den Angaben in Beispiel 1.

### Patentansprüche

1. Verfahren zur Herstellung von 1-Aminopropandiol-2,3 durch Umsetzung von Glycid mit Ammoniak, dadurch gekennzeichnet, dass man Glycid und flüssiges Ammoniak unter einem solchen Druck, dass das Ammoniak in flüssigem Zustand verbleibt, in Gegenwart einer geringen Menge Wasser, und zwar in einem Gewichtsverhältnis von Glycid zu Wasser von 1:0,05 bis 1:10, umsetzt.

2. Verfahren nach Anspruch 1, dadurch gekennzeichnet, dass man Glycid und flüssiges Ammoniak im Molverhältnis von 1:5 bis 1:20 einsetzt.

3. Verfahren nach Anspruch 1 und 2, dadurch gekennzeichnet, dass man Glycid und flüssiges Ammoniak im Molverhältnis von 1:10 bis 1:20 miteinander umsetzt.

4. Verfahren nach Anspruch 1–3, dadurch gekennzeichnet, dass man Glycid und flüssiges Ammoniak im Molverhältnis von 1:17 miteinander umsetzt.

5. Verfahren nach Anspruch 1–4, dadurch gekennzeichnet, dass man Glycid und Wasser in einem Gewichtsverhältnis von 1:1 einsetzt.

6. Verfahren nach Anspruch 1–5, dadurch gekennzeichnet, dass man die Reaktion unter einem Druck von 5 bis 150 bar, bevorzugt von 20 bis 90 bar, durchführt.

### Claims

1. Process for the production of 1-amino-propane-2,3-diol, by reacting glycidol with ammonia, characterised in that glycidol and liquid ammonia are reacted under a pressure such that the ammonia remains in liquid state, in the presence of a small quantity of water and in a weight ratio of glycidol to water of from 1:0.05 to 1:10.

2. Process according to claim 1, characterised in that glycidol and liquid ammonia are used in a mol ratio of from 1:5 bo 1:20.

3. Process according to claims 1 and 2, characterised in that glycidol and liquid ammonia are reacted together in a mol ratio of from 1:10 to 1:20.

4. Process according to claims 1 to 3, characterised in that glycidol and liquid ammonia are reacted together in a mol ratio of 1:17.

5. Process according to claims 1 to 4, characterised in that glycidol and water are used in a weight ratio of 1:1.

6. Process according to claims 1 to 5, characterised in that the reaction is carried out under a pressure of from 5 to 150 bars, preferably from 20 to 90 bars.

**Revendications**

1. Procédé de préparation de 1-amino-propandiol-2,3, par réaction du glycide avec l'ammoniac, procédé caractérisé en ce que l'on fait réagir le glycide et l'ammoniac liquide sous une pression telle que l'ammoniac reste à l'état liquide, en présence d'une faible quantité d'eau, et avec un rapport en poids du glycide à l'eau de 1:0,05 à 1:10.

2. Procédé selon la revendication 1, caractérisé en ce que l'on fait réagir le glycide et l'ammoniac liquide dans un rapport molaire de 1:5 à 1:20.

3. Procédé selon les revendications 1 et 2, caractérisé en ce que l'on fait réagir le glycide et l'ammoniac liquide dans un rapport molaire de 1:10 à 1:20.

4. Procédé selon les revendications 1 à 3, caractérisé en ce que l'on fait réagir le glycide et l'ammoniac liquide dans un rapport molaire et 1:17.

5. Procédé selon les revendications 1 à 4, caractérisé en ce que l'on met en œuvre le glycide et l'eau dans un rapport en poids de 1:1.

6. Procédé selon les revendications 1 à 5, caractérisé en ce que l'on effectue la réaction sous une pression de 5 à 150 bars, de préférence de 20 à 90 bars.